# EUROPEAN PATENT APPLICATION

(11) **EP 4 847 521 A1**
(43) Date of publication of application: **02.12.2026**
(21) Application number: 25745314.2
(22) Date of filing: 22.01.2025
(51) Int. Cl.: C07K 14/00, A61K 49/00

(54) **PEPTIDE-BASED PROBES FOR DIAGNOSIS OF DISEASE AND USES THEREOF**

(30) Priority: 26.01.2024 KR 20240012487
(71) Applicant: Nibec Co., Ltd., Chungcheongbuk-do 27816 (KR); Seoul National University R&DB Foundation, Seoul 08826 (KR)
(72) Inventor: CHUNG, Chong-Pyoung, Seoul 05618 (KR); PARK, Yoon Jeong, Seoul 08291 (KR); LEE, Jue-Yeon, Gwacheon-si, Gyeonggi-do 13831 (KR); JO, Beom Soo, Seoul 05817 (KR); YOON, Gook-Jin, Seoul 03080 (KR); LEE, Dong Woo, Seoul 08213 (KR)
(74) Representative: Mewburn Ellis LLP
(86) International application number: PCT/KR2025/001210
(87) International publication number: WO 2025/159488

(57) **Abstract**

The present invention relates to a probe in which a fluorescent dye-quencher is additionally bound to a fusion peptide to which a cell-penetrating peptide-biomarker binding functional peptide for diseases is bound and, more particularly, to a cell-penetrating peptide-bioactive peptide molecular probe which directly introduces a non-penetrating disease-sensitive important domain into a cell so as to penetrate a target cell and emit fluorescence when bound to a target material, thereby facilitating diagnosis and simultaneously inducing treatment. According to the present invention, compared to conventional staining-based histological disease diagnosis, diseases, such as inflammation, can be identified in a non-invasive and rapid manner, and thus the probe may be useful in diagnosing various inflammatory diseases or monitoring transplant-related inflammatory responses during *in vivo* transplantation. Also, the probe is applicable to clinical use without undergoing multiple steps and can be mass-produced, and thus, by using this approach, is useful in the development of drug delivery systems and treatment technologies.

## Description

### [Technical Field]

The present invention relates to a fusion peptide that includes a cell-penetrating peptide and a bioactive peptide, and is labeled with a fluorescent dye and a quencher, and the use thereof, and more specifically, to a probe designed such that, by further binding a fluorescent dye and a quencher to both termini of a fusion peptide formed by chemically binding a cell-penetrating peptide to a bioactive peptide, fluorescence is not emitted due to the quencher until the bioactive peptide reaches a target, and fluorescence is detected when the bioactive peptide binds to a target biomarker, and to a diagnostic composition containing the same, and a diagnostic method including the same.

### [Background Art]

Noninvasive and simultaneous imaging and therapy have been attempted and have achieved some success in the treatment of cancer and inflammatory diseases (Kim EM, et al., Nucl Med Biol 2009;36:371-8). Most of such imaging has relied on specific enzymatic activity predominantly expressed at targets for substrates previously conjugated to activatable probes (Akhatib B, et al., J Biol Chem 2013;288:19280-7). For example, in rheumatoid arthritis, a matrix metalloprotease (MMP)-3-specific substrate-modified polymer has been studied as a near-infrared fluorescence (NIRF) probe (Ryu JH, et al., Arthritis Rheum 2011;63:3824-32). The problem was that the accuracy thereof was recognized only after deterioration to the middle stage or later, at which this marker was present at a high concentration, and the probe was not specific to a single MMP. Rather than this, designing a probe based on protein-protein binding may be more advantageous for early detection. Such specific binding of one molecule to another molecule is a well-known biological phenomenon and many diagnostic and therapeutic techniques based thereon have been derived. Stable covalent bond-forming molecules between synthetic probes and specific sites of target proteins have many potential applications in biomedical science (Marquez BV, et al., Bioconjugate Chemistry 2012;16:1080-9). Among the more interesting examples are peptide ligands, the strongest of which generally bind to receptors with nanomolar dissociation constants, making them advantageous for confirming specificity (Ehrlich A, et al., Biochem Pharmacol 2013;86:1263-71).

The present inventors applied an E3 ubiquitin enzyme system as a protein-binding target. Ubiquitination is a natural proteasomal degradation reaction for balancing the removal process of aged proteins or abnormal proteins, and dysfunction or excessive action of ubiquitination leads to inflammation or disease states. In the ubiquitin proteasome system (UPS), ligation of ubiquitin is mediated by sequential actions of E1-E2-E3 enzymes (Tian M, Xie Q. J Integr Plant Biol 2013;55:54-63). Ubiquitin is activated by E1 and then the thioester bond is formed between the ubiquitin C-terminus and the cysteine of E1, thereby generating activated ubiquitin. Final binding between the ubiquitin C-terminus and a target is formed by an E3 ligase (Schulman BA, Harper JW. Nat Rev Mol Cell Biol 2009;10:319-31). Most studies target E3 ligase as a key molecule in many diseases associated with UPS (Wang Z, et al., Neoplasia 2013;15:1028-35). Two major classes of E3 ligase are RING-type and HECT-type E3, and C2-WW-HECT E3 has been most extensively studied. Subfamilies of E3 generally consist of an N-terminal C2 domain, and 2 to 4 WW domains for substrate interaction. Smurf-1, which is an example of an E3 ubiquitin ligase having domains for substrate interaction and a C-terminal HECT domain for maintaining E3 activity, is a HECT-class ubiquitin ligase that specifically binds to Smad proteins to promote proteasome-mediated degradation of Smad, and is a marker frequently observed in inflammatory diseases (Sangadala S, et al., Biochemistry 2006;281:17212-9). Smads, particularly Smad 1/5/8, are also associated with osteogenesis. Overexpression of Smurf is significant in most inflammatory diseases such as rheumatoid arthritis, periodontitis, osteoporosis, other bone resorption diseases, and cancer. In these inflammatory diseases, it is clear that osteogenesis is poor due to degradation of Smad proteins. The role of E3-ubiquitin is to induce proteasomal degradation based on specific binding to a target protein. The present inventors designed a peptide motif as a therapeutic tool for blocking proteasomal degradation of Smads by binding E3 ligase in cells and tissues overexpressing Smurf-1. LIM mineralization protein (LMP)-1 is a recently identified intracellular protein and has been shown to stimulate osteoblast differentiation and generate mineralized nodules in murine calvarial osteoblasts *in vitro* (Boden SD, et al., Endocrinology 1998;139:5125-34). Although the exact mechanism or key region of the osteogenic action of LMP-1 is not known, LMP-1 has been shown to strongly bind to Smurf-1 (Sangadala S, et al., Proteins 2007;68:690-701).

The present inventors hypothesized that a specific domain sequence of LMP-1 could be prepared by peptide synthesis and could mimic the full activity of LMP-1, and also hypothesized that the domain sequence of LMP-1 would strongly bind to Smurf, which is an inflammatory marker. The generated peptide should satisfy three essential requirements: (i) the peptide should maintain the same chemical and physical properties as LMP-1, (ii) the peptide should have a well-defined Smurf-1 binding affinity (i.e., greater than the binding affinity between Smurf-1 and Smad), and (iii) the sequence should be delivered into cells and should be labeled with a fluorescent dye capable of monitoring intracellular localization and binding of Smurf-1. When target binding can alter the peptide conformation, detection may be achieved through fluorescence resonance energy transfer (FRET) imaging. That is, a fluorescent dye and a quencher dye are bound to respective termini of the peptide. There is no fluorescence, but conformational change may increase the distance between the two dyes. According to the present invention, the peptide probe was conjugated to the sequence together with BHQ-1 and a fluorescent dye and exhibited fluorescence inactivity until reaching Smurf-1, and the peptide probe was switched to an active state, as the distance between the fluorescent dye and quencher increased after binding between Smurf-1 and a Smurf1-binding peptide (SBP) sequence, thereby compensating for interference. As a result, it was confirmed that the peptide motif, referred to as a peptide probe, can provide a convenient, rapid, specific, and sensitive means for monitoring an analyte and reporting the presence of a specific target substrate. Based on this finding, the present invention has been completed.

The above information disclosed in this Background section is provided only for enhancement of understanding of the background of the invention, and therefore it may not include information that forms the prior art that is already obvious to those skilled in the art.

### [Disclosure]

It is one object of the present invention to provide a probe in which a fluorescent dye and a quencher are labeled on a fusion peptide comprising a cell-penetrating peptide and a bioactive peptide bound to each other, to diagnose a disease rapidly in a noninvasive manner.

It is another object of the present invention to provide a composition for diagnosing an inflammatory disease, containing the probe.

It is another object of the present invention to provide a method of diagnosing an inflammatory disease including the probe and a method of providing information for diagnosing an inflammatory disease including the probe.

In accordance with one aspect of the present invention, the above and other objects can be accomplished by the provision of a probe for detecting a biomarker, comprising a fusion peptide comprising a cell-penetrating peptide and a bioactive peptide bound to each other, and a fluorescent dye and a quencher bound to the fusion peptide.

In accordance with another aspect, provided is a composition for diagnosing an inflammatory disease, containing the probe.

In accordance with another aspect, provided is a method of diagnosing an inflammatory disease and a method of providing information for diagnosis, each method including treating a sample isolated from a subject with the probe.

### [Description of Drawings]

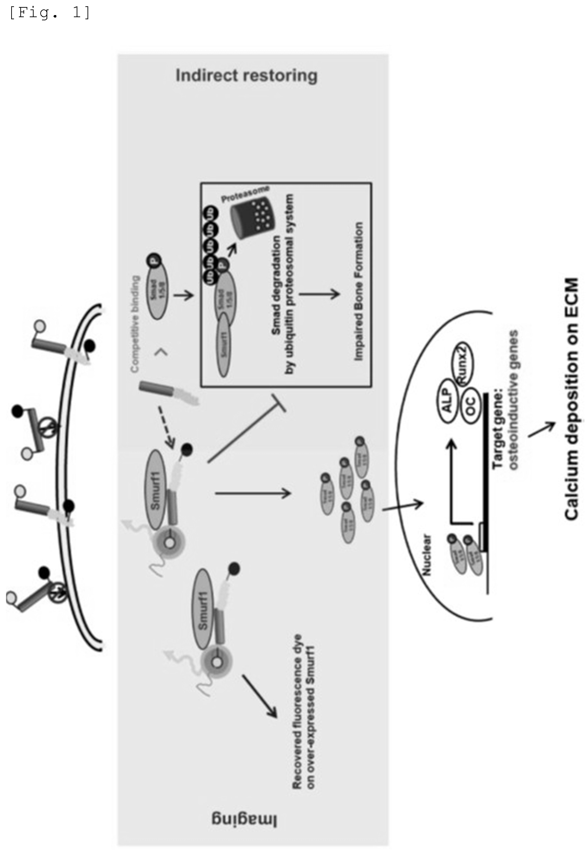

FIG. 1 is a schematic diagram illustrating the concept of real-time molecular imaging by a peptide probe based on a ubiquitin proteasome system (UPS), and application thereof to diagnosis and therapy.

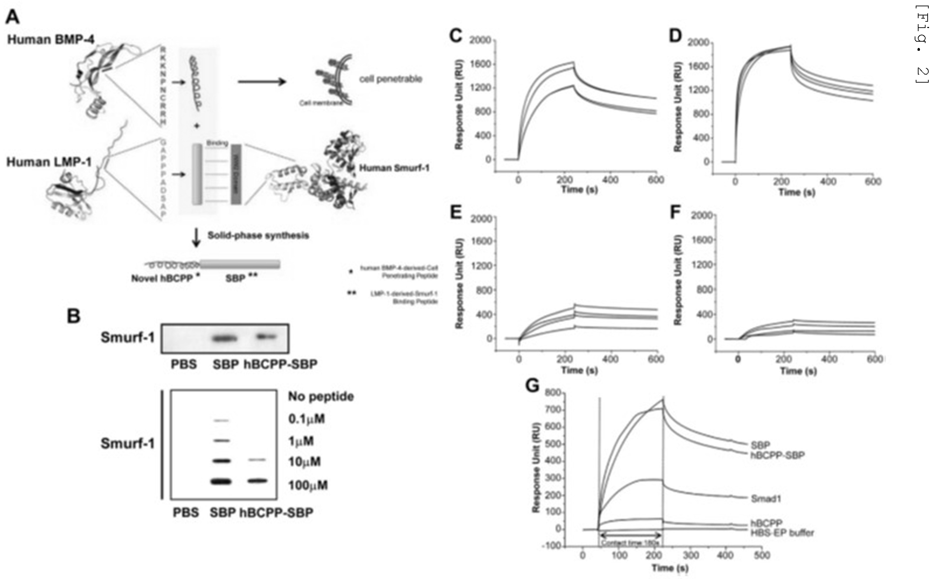

FIG. 2 is (A) a schematic diagram illustrating peptide design and production using Fmoc solid-phase peptide synthesis, shows (B) results of evaluation of binding of SBP and hBCPP-SBP including a WW domain interaction site to Smurf-1 using slot blot analysis, and are sensorgrams confirming interactions between (C) SBP and Smurf-1, (D) hBCPP-SBP and Smurf-1, (E) human recombinant protein Smad 1 and Smurf-1, and (F) hBCPP and Smurf-1, wherein concentrations from the top of each graph are 1000, 4000, 6000, and 8000 nM, and shows (G) results of SPR analysis for specific binding between 1 µM of each peptide and immobilized Smurf-1.

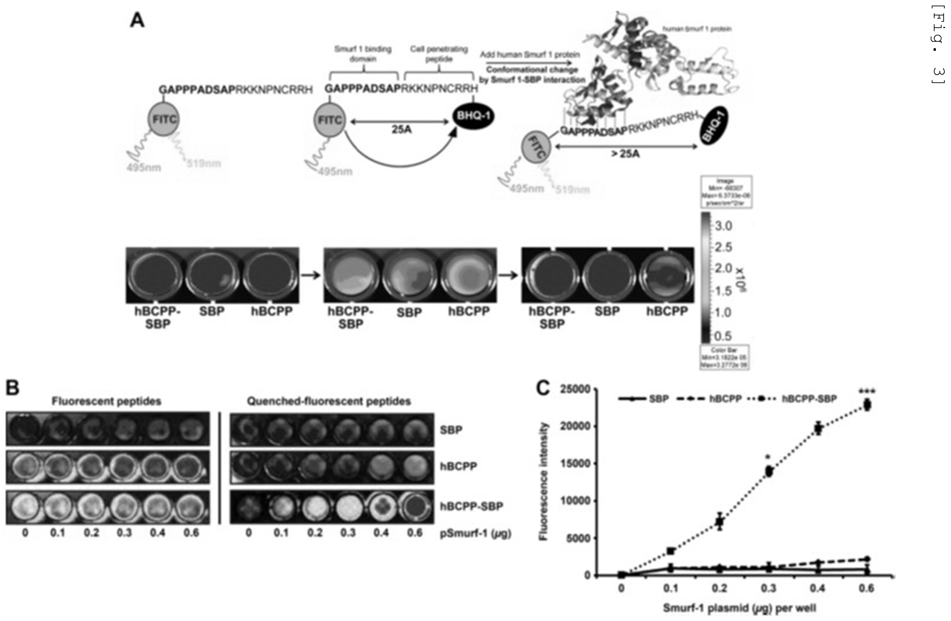

FIG. 3 is (A) a schematic diagram illustrating fluorescence imaging of a fluorescence-labeled peptide according to structural change upon binding to a target marker (light emission, left panel), a BHQ-1-conjugated fluorescence-labeled peptide (middle panel), and a re-fluoresent peptide using Smurf-1 protein (light re-emission, right panel), shows (B) results of treating Smurf-1-overexpressing hMSC with a fluorescence-quenched peptide at various doses (fluorescence images were detected at 488 nm using LAS3000), and shows (C) fluorescence intensity quantified using a fluorometer. Data are presented as mean ± standard error of the mean (*p < 0.05, ***p < 0.01).

### [Best Mode]

Unless defined otherwise, all technical and scientific terms used herein have the same meanings as appreciated by those skilled in the field to which the present invention pertains. In general, the nomenclature used herein is well-known in the art and is ordinarily used.

The present invention relates to a peptide probe that can be more usefully applied to diagnosis of diseases including inflammation, based on an intracellular delivery technique that enables non-permeable bioactive substances, such as peptides or drugs bound thereto, to penetrate into cells using a cell-penetrating peptide.

A cell-penetrating peptide was chemically conjugated so that non-permeable bioactive substances, such as peptides or drugs to be introduced, can be rapidly and safely delivered into cells by *in vivo* and *in vitro* treatment, that is, directly introduced into cells without an endocytosis process, which is a conventional intracellular uptake method, and a peptide probe labeled with a fluorescent dye and a quencher on the fusion peptide was produced. Since, after the probe penetrates into target cells by the cell-penetrating peptide, the distance between the quencher and the fluorescent dye increases and light is emitted only when the bioactive peptide binds to a biomarker, molecular diagnosis can be easily performed even at low concentrations, which indicates that the probe is useful for diagnosis of diseases expressing a target biomarker.

Accordingly, in one aspect, the present invention is directed to a probe for detecting a biomarker, comprising a fusion peptide comprising a cell-penetrating peptide and a bioactive peptide bound to each other, and a fluorescent dye and a quencher bound to the fusion peptide.

As used herein, the term "probe" refers to a peptide- or protein-based probe to determine the presence or absence of a specific biomarker, wherein the probe has cell permeability based on the cell-penetrating peptide, includes a bioactive peptide that specifically binds to a specific biomarker, and is conjugated with the fluorescent dye and the quencher within a distance at which the fluorescent dye can be quenched by binding of the quencher.

As used herein, the term "biomarker" refers to molecular information based on a single molecule or a pattern of molecules derived from DNA, RNA, metabolites, proteins, and protein fragments, and is an indicator that enables detection of bodily changes caused by the influence of genetic or epigenetic changes in a living organism.

As used herein, the term "cell-penetrating peptide" is used interchangeably with "cell-penetrating domain" and "protein transduction domain (PTD)" and refers to a permeable functional peptide capable of delivering peptides, drugs, or drug-containing particles into the cytoplasm or nucleus of a cell. The cell-penetrating peptide may form a covalent bond with oligonucleotides, peptides, proteins, oligosaccharides, polysaccharides, or nanoparticles, and may deliver these materials into cells without requiring a separate receptor, carrier, or energy.

In the present invention, the cell-penetrating peptide may include an amino acid sequence of 5 to 15 amino acids and contain 40 to 70% in total of at least one amino acid selected from the group consisting of arginine (R), lysine (K), and histidine (H), but is not limited thereto.

In the present invention, the cell-penetrating peptide may include at least one amino acid sequence selected from the group consisting of SEQ ID NO: 1 to SEQ ID NO: 12:

H4S (SEQ ID NO: 1: SSRKKNPNCRRH), H4Q (SEQ ID NO: 2: QRARKKNKNCRRH), HBD-3P (SEQ ID NO: 3: CSTRGRKCCRRKK), H2 (SEQ ID NO: 4: HKREKRQAKHKQRKR), H3 (SEQ ID NO: 5: KSKNKKKQRKGPHRK), H3B (SEQ ID NO: 6: KPRPGRKDRRKK), H4-1 (SEQ ID NO: 7: RRRRAKRSPKHHS), H6 (SEQ ID NO: 8: SRRRQQSRNR), H8 SEQ ID NO: 9: RAVRPLRRRQPKKS), H4C (SEQ ID NO: 10: CSSRKKNPNCRRH), H5C (SEQ ID NO: 11: CSSRKKNKNCPRRH) and H6C (SEQ ID NO: 12: CSSRKKNPNCPRRH).

H4Q is a peptide derived from human bone morphogenetic protein (BMP)-4, HBD-3P is a peptide derived from human beta defensin, H2 is a peptide derived from human bone morphogenetic protein (BMP)-2, H3 and H3B are peptides derived from human bone morphogenetic protein (BMP)-3, H4-1 is a peptide obtained by partially modifying a peptide derived from human bone morphogenetic protein (BMP)-4, H6 is a peptide derived from human bone morphogenetic protein (BMP)-6, and H8 is a peptide derived from human bone morphogenetic protein (BMP)-8.

The amino acids constituting the cell-penetrating peptide may be of an L-form or a D-form in consideration of *in vivo* stability.

In the present invention, peptides or peptide analogs other than the above-described peptides may also be used as long as they are capable of penetrating a cell membrane. Similarly, the fusion peptide of the cell-penetrating peptide and the bioactive peptide according to the present invention may also be produced using conventional non-human-derived cell-penetrating functional domains, for example, TAT or arginine-derived peptides. For improved biocompatibility, embodiments of the present invention utilize human-derived peptides. However, the present invention is also applicable to conventional viral or non-human-derived peptides.

As used herein, the term "bioactive peptide" is used interchangeably with "bioactive protein", "bioactive domain", "biomarker conjugate", and "biomarker-binding sequence" and may be a peptide containing an amino acid sequence that specifically binds to a specific biomarker and has anti-inflammatory activity, antimicrobial activity, anticancer activity, tissue regeneration activity, or bone regeneration-inducing activity.

In the present invention, the bioactive peptide may include at least one amino acid sequence selected from the group consisting of SEQ ID NO: 13 to SEQ ID NO: 16:

SEQ ID NO: 13 (GAPPPADSAP), SEQ ID NO: 14 (PPGY), and SEQ ID NO: 15 (PPAY), which are bone differentiation- or bone regeneration-inducing sequences, and SEQ ID NO: 16 (anti care peptide: TRGRKCCRRKK), which is an anti-inflammatory sequence.

In the present invention, the fusion peptide of the cell-penetrating peptide and the bioactive peptide may be produced by chemical synthesis using a peptide synthesizer, wherein a bone differentiation-inducing sequence or a bioactive domain is sequentially chemically synthesized at the C-terminus of a protein transduction domain (PTD) having intracellular permeability, such that the fusion peptide may be synthesized in the order of "N-terminus-protein transduction domain-bioactive domain-C-terminus" or in the order of "N-terminus-bioactive domain-protein transduction domain-C-terminus". The bioactive domain is mainly used for binding to a biomarker of a disease. The bioactive domain may also have pharmacologically active functions, for example, not only bone differentiation-inducing activity but also the ability, as a substance that regulates gene expression and physiological functions *in vitro* or *in vivo*, to correct abnormal pathological conditions caused by deficiency or excessive secretion of substances involved in functional regulation *in vivo*, and may be of an L-form or D-form in consideration of *in vivo* stability.

In the present invention, the fusion peptide may be applied to detection of biomarkers in cells and *in vivo* and to diagnosis of diseases by covalent binding with a fluorescent dye and a quencher. At this time, the fluorescent dye or quencher may be covalently bound to the N-terminus or C-terminus of the fusion peptide, and preferably may be covalently bound by further attaching cysteine to a terminus of the cell-penetrating peptide or the bioactive peptide.

In addition, a complex of the fusion peptide with the fluorescent dye and the quencher may be produced by inducing chemical bonding using a crosslinker. When chemical bonding is induced using a crosslinker, complex formation by the crosslinker is facilitated because the N-terminus of the cell-penetrating peptide has a free amino group. Crosslinkers usable in the present invention include 1,4-bis-maleimidobutane (BMB), 1,11-bis-maleimidotetraethyleneglycol (BM[PEO]4), 1-ethyl-3-[3-dimethylaminopropyl] carbodiimide hydrochloride (EDC), succinimidyl-4-[N-maleimidomethylcyclohexane-1-carboxy-[6-amidocaproate]] (SMCC) and sulfonated salts thereof (sulfo-SMCC), succinimidyl 6-[3-(2-pyridyldithio)-propionamido] hexanoate (SPDP) and sulfonated salts thereof (sulfo-SPDP), m-maleimidobenzoyl-N-hydroxysuccinimide ester (MBS) and sulfonated salts thereof (sulfo-MBS), succinimidyl[4-(p-maleimidophenyl)butyrate] (SMPB) and sulfonated salts thereof (sulfo-SMPB), but are not limited thereto. In particular, since labeling only a fluorescent dye makes accurate diagnosis difficult due to non-specific fluorescence expression, application of a quencher, which is essential for a fluorescence resonance energy transfer (FRET) designed to fluoresce only when the bioactive domain binds to a marker protein of a disease, is required.

In the present invention, the fluorescent dye may be selected from the group consisting of fluorescein, fluorescein isothiocyanate (FITC), fluorescein chlorotriazinyl, rhodamine green, rhodamine red, tetramethylrhodamine, Oregon Green, Alexa Fluor, FAM, JOE, ROX, HEX, Texas Red, TET, TRITC, TAMRA, cyanine-based dyes, and thiadicarbocyanine, and the quencher may be selected from the group consisting of Dabcyl, TAMRA, Eclipse, DDQ, QSY, Blackberry Quencher, Black Hole Quencher (BHQ), Qxl, Iowa Black FQ, Iowa Black RQ, and IRDye QC-1, but is not limited thereto. Preferably, the fluorescent dye may be FITC and the quencher may be BHQ-1, but is not limited thereto.

In another aspect, the present invention is directed to a composition for diagnosing an inflammatory disease, containing a probe for detecting a biomarker.

As used herein, the term "diagnosis" means accurately determining the condition of a subject with respect to a specific disease or disorder, and includes determining the disease name, cause, disease type, severity, detailed aspects of symptoms, and presence or absence of complications. For example, the condition of a subject with respect to a specific disease or disorder is used in a broad sense to include susceptibility to a specific disease or disorder, determination of a disease currently suffered by the subject, identification of characteristics of the disease such as prognosis of the subject, identification of a disease state, determination of a disease stage, or prediction of cancer susceptibility and responsiveness to treatment, obtaining a basis for appropriate treatment according to the disease and condition of the patient, such as confirmation of the condition of the subject to determine the therapeutic effect of a specific drug, and further prediction and confirmation of recurrence in a subject cured of a specific disease or disorder.

In the present invention, the inflammatory disease may be selected from the group consisting of bone disease, fibrosis, periodontitis, rheumatoid arthritis, inflammatory bowel disease, inflammatory response to biomaterial implantation, asthma, dermatitis, psoriasis, and cancer, but is not limited thereto.

In the present invention, the bone disease may be selected from the group consisting of osteoporosis, osteoarthritis, osteitis, osteogenesis imperfecta, hypercalcemia, osteomalacia, Paget's disease, bone loss caused by cancer, and osteonecrosis, but is not limited thereto.

In another aspect, the present invention is directed to a kit for diagnosing an inflammatory disease, including the probe for detecting a biomarker.

In another aspect, the present invention is directed to a method of diagnosing an inflammatory disease and a method of providing diagnostic information, each method including contacting a sample isolated from a subject with the probe for detecting a biomarker.

In the aspects of the present invention, definitions and embodiments of terms not described herein may share the same features as those described in the context of the composition for diagnosing an inflammatory disease, unless otherwise specified.

Hereinafter, the present invention will be described in more detail with reference to examples. However, it will be obvious to those skilled in the art that these examples are provided only for illustration of the present invention and should not be construed as limiting the scope of the present invention.

### Example 1: Synthesis of Fusion Peptide

A fusion peptide comprising, from the N-terminus, GAPPPADSAP (SEQ ID NO: 13), which is a bone differentiation-inducing sequence derived from LMP1, and H4S (SSRKKNPNCRRH: SEQ ID NO: 1) as a PTD, was synthesized by an Fmoc solid-phase chemical synthesis method using a peptide synthesizer (FIG. 1). Synthesis was performed using Rink resin (0.075 mmol/g, 100 to 200 mesh, 1% DVB crosslinking) conjugated with Fmoc (9-fluorenylmethoxycarbonyl) as a blocking group. After 50 mg of Rink resin was loaded into the synthesizer and the resin was swelled with DMF, a 20% piperidine/DMF solution was used to remove the Fmoc group. From the C-terminus according to the sequence, 0.5 M amino acid solution (solvent: DMF), 1.0 M DIPEA (solvent: DMF & NMP), and 0.5 M HBTU (solvent: DMF) were added in amounts of 5, 10, and 5 equivalents, respectively, and reacted for 1 to 2 hours under a nitrogen stream. After each deprotection and coupling step, washing with DMF and NMP was performed twice each. After final amino acid was coupled, deprotection was also performed to remove the Fmoc group.

Synthesis was confirmed using a ninhydrin test. After the test and completion of synthesis, the resin was dried with THF or DCM, and a TFA cleavage cocktail was added at a ratio of 20 mL per 1 g of resin, followed by shaking for 3 hours. Then, the resin and the cocktail containing the dissolved peptide were separated by filtration. After the filtrate was removed using a rotary evaporator, cold ether was added, or alternatively, excess cold ether was directly added to the TFA cocktail solution containing the dissolved peptide to crystallize the peptide into a solid phase, followed by centrifugation. At this time, the TFA cocktail was completely removed through repeated washing with ether and centrifugation. The peptide thus obtained was dissolved in distilled water and freeze-dried.

Fusion peptide: NH₂-GAPPPADSAP-SSRKKNPNCRRH-C-COONH₂ (SEQ ID NO: 17)
The synthesized peptide sequence was cleaved from the resin, washed, freeze-dried, and then separated and purified by liquid chromatography. The molecular weight of the purified peptide was measured by MALDI analysis.

Comparative Example 1: H4S (SSRKKNPNCRRH: SEQ ID NO: 1) as PTD
The peptide was synthesized by an Fmoc solid-phase chemical synthesis method using a peptide synthesizer.

Comparative Example 2: Inflammatory marker-binding sequence derived from LMP1 (GAPPPADSAP: SEQ ID NO: 13)
The peptide was synthesized by an Fmoc solid-phase chemical synthesis method using a peptide synthesizer.

### Example 2: Confirmation of Binding Affinity Between Fusion Peptide and Inflammatory Marker Smurf1

In order to chemically confirm the binding affinity between the fusion peptide synthesized by the method of Example 1, and Smurf1, human-derived Smurf1 protein acting as a ligand in this experiment was purchased from OriGene Technologies (Rockville, MD, USA). 100 mg/mL of the protein was immobilized on a gold-coated surface of a CM5 chip (Cytiva, Sweden), in which amino groups were bound to the surface, using an EDC/NHS kit (BIACORE AB, Sweden) (the above experimental method may alternatively use "Immobilization" embedded in BIACORE T100 (BIACORE AB, Sweden) software used by the present inventors). At this time, in order to find an appropriate pH condition under which the protein can bind to amino groups of the CM5 chip, a method referred to as "pH scouting" was performed to determine a pH condition. For the "pH scouting" experimental method, the method embedded in BIACORE T100 (BIACORE AB, Sweden) software used by the present inventors may alternatively be used. After immobilizing the ligand under the pH condition determined by this method, analytes (the fusion peptide synthesized in Example 1, the cell-penetrating peptide of Comparative Example 1 used as a negative control, and the inflammatory marker-binding sequence of Comparative Example 2 used as a positive control) were each flowed over the CM5 chip at 10 mM, and binding affinity was measured (the above experimental method may alternatively use "binding analysis" embedded in BIACORE T100 (BIACORE AB, Sweden) software used by the present inventors).

As a result, as shown in FIG. 2A, Comparative Example 2, which binds to the WW domain within the Smurf1 protein sequence and has bone differentiation capability, exhibited a binding affinity to Smurf1 of 750 RU (resonance unit; unit of binding affinity measurement), the fusion peptide exhibited 600 RU, and the cell-penetrating peptide of Comparative Example 1 used as a negative control exhibited 100 RU (FIGs. 2B to 2G). These results indicate that the fusion peptide has a bone differentiation-inducing sequence and that no significant damage was caused to the bioactive sequence (a region capable of exhibiting functionality by binding to Smurf1 *in vivo*) during synthesis.

In osteoarthritis or other inflammatory diseases, Smurf1, as an inflammatory factor that interferes with tissue maintenance or regeneration, strongly binds to its counter protein SMAD and subsequently promotes ubiquitination that degrades the corresponding protein, thereby irreversibly interfering with cellular regeneration. The present inventors determined that as overexpression of Smurf1 increases, the state of the inflammatory disease becomes more severe.

The apparent association constants (KA) for LMP-1-derived Smurf1-binding peptide (SBP), cell-penetrating SBP fusion peptide (hBCPP-SBP), and cell-penetrating peptide (hBCPP) were calculated to be 1.036E+7(M-1), 1.237E+7(M-1), and 4.129E+5(M-1), respectively. Accordingly, the apparent dissociation constants (KD = 1/KA) for SBP, hBCPP-SBP, and hBCPP were 96.6 nM, 80.8 nM, and 2422 nM, respectively. In contrast, the apparent dissociation constant of Smad-1, which is known to strongly bind to Smurf-1 during ubiquitination, is 298 nM (Table 1).

**[Table 1]**

| Binding parameters of Smurf-1 for SBP and hBCPP-SBP | | | | | |
|---|---|---|---|---|---|
| Kinetic parameters of the binding of Smurf1 to SBP, hBCPP-SBP, hBCPP, and Smad1. | | | | | |
| Interacting molecules | KD (M) | KA (1/M) | Ka (1/MS) | Kd (1/s) | Rmax (RU) |
| Smurf-1-SBP | 9.657E-8 | 1.036E+7 | 6671 | 6.442E-4 | 1371.5 |
| Smurf-1-hBCPP-SBP | 8.080E-8 | 1.237E+7 | 7321 | 5.916E-4 | 1475.0 |
| Smurf-1-hBCPP | 2 422E-5 | 4.129E+5 | 2447 | 0.005927 | 22.4 |
| Smurf-1-Smad-1 | 2.980E-7 | 3.360E+6 | 3548 | 1.057E-3 | 348.6 |

Accordingly, it can be concluded from the binding constants that there is a stronger interaction between SBP and Smurf-1 than between Smad-1 and Smurf-1. Therefore, LMP-1 may compete with Smad1/5/8 for binding to Smurf-1 and inhibit targeting of ubiquitination. This relatively high affinity increases the likelihood that the Smurf-1 interaction has biological significance. These results are consistent with the binding analysis and demonstrate that hBCPP-SBP identified in the present study is an important domain of LMP for inhibiting Smurf-1 and is further activated by addition of the human-derived cell-penetrating peptide (hBCPP) by the present inventors.

In addition, this function suggests that ubiquitination can be regulated by a cell-permeable target-binding peptide probe, which supports the potential thereof as a therapeutic agent. As can be seen from FIG. 2, Smurf1 strongly binds to SMAD and induces proteasomal degradation by ubiquitination and, by binding of Smurf1 to the peptide probe, the degradation process was inhibited and degradation of SMAD was halted.

### Example 3: Simultaneous Imaging by Fusion Peptide Probe in Cells Overexpressing Smurf-1

In order to investigate the binding interaction between the bioactive peptide (SBP) sequence and Smurf-1 by a quenching strategy referred to as fluorescence resonance energy transfer (FRET), the fusion peptide was further modified with an activatable fluorescent dye. BHQ-1 can quench fluorescence of fluorescein isothiocyanate (FITC) with high efficiency. In the present study, fluorescence of two peptides (hBCPP and SBP) was inactive because the fluorescent dye and BHQ were in close proximity, but fluorescence activity appeared after treatment with Smurf-1 because BHQ was separated due to binding between Smurf-1 and SBP (FIG. 3A). Fluorescence intensity was proportional to the concentration of Smurf-1, indicating that the peptide probe of the present invention is useful for diagnosis of inflammatory diseases (FIGs. 3B and 3C). This is likely the result of a structural change in the peptide caused by relatively large Smurf-1. In order to examine the following results *in vitro*, FRET analysis was performed on human osteosarcoma (HOS) cells, which are positive for Smurf-1 expression, and mouse fibroblasts (NIH3T3), which are negative for Smurf-1 expression (FIG. 3). HOS cells treated with the quenched hBCPP-SBP probe restored a fluorescence signal, whereas NIH3T3 cells did not. Meanwhile, neither HOS nor NIH3T3 exhibited fluorescence when the cell-penetrating functional peptide was not linked, that is, in cells treated with the target-binding peptide SBP, because SBP could not penetrate the cell membrane by itself. In addition, since hBCPP, which is a cell-penetrating functional peptide, does not bind to Smurf-1, recovery of a fluorescence signal was not observed in cells treated with this peptide.

### [Industrial Applicability]

The diagnostic method according to the present invention is noninvasive and can identify a disease, for example, an inflammatory condition, relatively rapidly as compared with conventional staining-based histological diagnosis, and thus can be usefully employed in diagnosis of various inflammatory diseases or monitoring of transplantation-related inflammatory responses during *in vivo* implantation. In addition, since the diagnostic method can be clinically applied without multiple steps and can be mass-produced, it is useful for development of diagnostic drug delivery systems and therapeutic technologies using the same.

Although specific configurations of the present invention have been described in detail, those skilled in the art will appreciate that this description is provided to set forth preferred embodiments for illustrative purposes and should not be construed as limiting the scope of the present invention. Therefore, the substantial scope of the present invention is defined by the accompanying claims and equivalents thereto.

### [Sequence Listing Free Text]

An electronic file is attached.

## Claims

1. A probe for detecting a biomarker, comprising:
a fusion peptide comprising a cell-penetrating peptide and a bioactive peptide bound to each other; and
a fluorescent dye and a quencher bound to the fusion peptide.

2. The probe according to claim 1, wherein the cell-penetrating peptide comprises an amino acid sequence of 5 to 15 amino acids and contains 40 to 70% in total of at least one amino acid selected from the group consisting of arginine (R), lysine (K), and histidine (H).

3. The probe according to claim 2, wherein the cell-penetrating peptide comprises at least one amino acid sequence selected from the group consisting of SEQ ID NO: 1 to SEQ ID NO: 12.

4. The probe according to claim 1, wherein the bioactive peptide comprises at least one amino acid sequence selected from the group consisting of SEQ ID NO: 13 to SEQ ID NO: 16.

5. A composition for diagnosing an inflammatory disease, comprising the probe according to any one of claims 1 to 4.

6. The composition according to claim 5, wherein the inflammatory disease is selected from the group consisting of bone disease, fibrosis, periodontitis, rheumatoid arthritis, inflammatory bowel disease, inflammatory response to biomaterial implantation, asthma, dermatitis, psoriasis, and cancer.

7. The composition according to claim 6, wherein the bone disease is selected from the group consisting of osteoporosis, osteoarthritis, osteitis, osteogenesis imperfecta, hypercalcemia, osteomalacia, Paget's disease, bone loss caused by cancer, and osteonecrosis.

8. A kit for diagnosing an inflammatory disease, comprising the probe according to any one of claims 1 to 4.
